# EUROPEAN PATENT SPECIFICATION

(11) **EP 1 587 517 B1**
(45) Date of publication and mention of the grant of the patent: **24.10.2007**
(21) Application number: 04701010.3
(22) Date of filing: 09.01.2004
(51) Int. Cl.: A61K 31/53, A61K 47/10, A61K 47/18, A61K 47/32, A61K 9/08, A61P 33/02

(54) **ANTI-PROTOZOAL COMPOSITIONS COMPRISING DICLAZURIL**
DICLAZURILHALTIGE ANTIPROTOZOENMITTEL
COMPOSITIONS ANTI-PROTOZOAIRES COMPRENANT DU DICLAZURIL

(30) Priority: 16.01.2003 WO PCT/EP03/00398
(43) Date of publication of application: 26.10.2005
(73) Proprietor: JANSSEN PHARMACEUTICA N.V., 2340 Beerse (BE)
(72) Inventor: DE SPIEGELEER, Bart, B-9000 Gent (BE); DOSOGNE, Hilde, B-9080 Lochristi (BE)
(74) Representative: Verberckmoes, Filip Gerard
(86) International application number: PCT/EP2004/000147
(87) International publication number: WO 2004/062673

(56) References cited:
- WO-A-98/43644
- US-A- 4 826 842
- US-A- 5 883 095

## Description

The present invention relates to compositions suitable for oral, transdermal or parenteral (*e.g*. intranasal, intramuscular, subcutaneous or intravenous) administration, wherein the composition is comprised of at least one anti-protozoal agent dissolved in a mixture of an alcohol based solvent-system, an emulsifier-system and a base-system. Also provided is a method for preparing said anti-protozoal compositions and their use in the treatment or prevention of protozoal infections in warm-blooded animals, including humans.

Protozoal parasites cause a variety of clinical disease manifestations in warm-blooded animals with in extreme case mortality. One family of drugs currently used for the treatment of protozoal infections is the triazine-based anticoccidial agents (*e.g*. triazinediones and triazinetriones), in particular diclazuril. These triazine-based anticoccidial agents are used as oral suspensions in veterinary medicine as well as have been tried experimentally in the treatment of cryptosporidiosis in human patients suffering from acquired immune deficiency syndrome (AIDS).

Bioavailability of the anti-protozoal agent diclazuril for the host is considered very poor. This very low oral bioavailability is related due to its extremely low aqueous solubility, with saturation concentrations below 1 µg/L in water. The solubility in most organic solvents is also low, except in dimethyl sulphoxide (48 g/litre), *N,N-*dimethylformamide (32.6 g/litre) and tetrahydrofuran (8.2 g/litre). Solutions of diclazuril in these organic solvents have been described in WO-00/19964; however, with the inherent drawback of solvent toxicity and of precipitation when diluted with aqueous systems as occurs in the *in vivo* situation after parenteral or oral administration.

The solubility of diclazuril in aqueous solutions can be increased by converting diclazuril into its base addition salt, e.g. its sodium salt. However it can be shown that sodium diclazuril in an aqueous solution is unstable and quickly starts to degrade into its keto-degradant having the following structure :

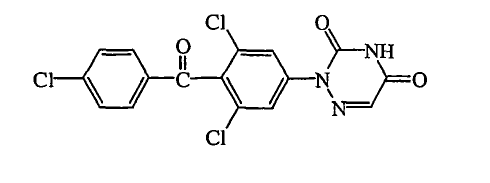

Many attempts have been made to find a therapeutically effective treatment for protozoal diseases, with limited and variable success, *inter alia* due to poor absorption of the orally formulated compounds. Hence there is a need for compositions comprising an anti-protozoal agent, in particular diclazuril, that combine a good bio-availability with good stability when such compositions are diluted with water.

The present invention satisfies the need in the art by providing a composition comprising diclazuril dissolved in a mixture comprising of an alcohol based solvent-system, an emulsifier-system and a base-system to give compositions that have a good bio-availability. Furthermore by selecting the specific alcohol based solvent-system, emulsifier-system and base-system the compositions of the present invention can be tailored for oral, parenteral or transdermal administration. The choice of the alcohol based solvent-system, emulsifier-system, base-system and concentration of the anti-protozoal agent dissolved therein will vary depending upon the choice of the specific anti-protozoal agent, the desired administration route, the species being treated and the sensitivity of the protozoal parasite towards such anti-protozoal agent. ,

The compositions provided by the invention eliminate the use of solvents with a relatively high toxicity profile such as DMSO, DMF and THF and which upon dilution with aqueous systems can cause precipitation of the active drug substance. Moreover, the present compositions are demonstrated to be stable upon dilution with aqueous systems such as artificial gastric fluid and artificial intestinal fluid.

The current compositions use alcohol based solvent-systems with low toxicity. They are designed to withstand precipitation upon dilution with aqueous systems, thereby reducing the risk of low and variable bioavailability as well as of local irritation after parenteral administration. In contrast to the aqueous formulations comprising the anti-protozoal agent - in particular diclazuril - in its base addition salt form, the current formulations have a significantly enhanced stability profile.

The anti-protozoal agent for use in the compositions of the present invention is the triazine-based anticoccidial agent diclazuril. The chemical structure of this triazine-based compound is shown below :

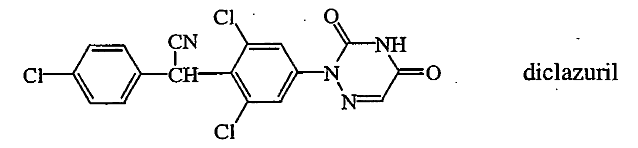

Wherever the term "diclazuril", is used, it is meant to include said compound both in its base form or in its acid addition or base addition salt form. The acid addition salts can conveniently be obtained by treating the base form with an appropriate inorganic or organic acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (*i.e*. butanedioic acid), maleic, fumaric, malic, tartaric, citric, methanesulfonic, ethanesulfonic, benzenesulfonic, *p*-toluenesulfonic, cyclamic, salicylic, *p*-aminosalicylic, pamoic and the like acids. The base additional salts can be conveniently obtained by treating the base form with appropriate organic and inorganic bases. Appropriate base salt forms comprise, for example, the ammonium salts, the alkali and earth alkaline metal salts, e.g. the lithium, sodium, potassium, magnesium, calcium salts and the like, salts with organic bases, e.g. the benzathine, *N*-methyl-D-glucamine, hydrabamine salts, and salts with amino acids such as, for example, arginine, lysine and the like. The term salt form as used hereinabove also comprises the solvates which the said compounds are able to form. Examples of such solvates are e.g., the hydrates, alcoholates and the like.

The **alcohol based solvent-system** comprises one or more alcohols. Said alcohols are defined as :
- lower alcohols comprising from 1 to 8 carbon atoms and more particularly from 2 to 6 carbon atoms, such as methanol, ethanol, propanol, isopropanol, butanol, isobutanol, *tert*-butanol, pentanol, hexanol, benzylalcohol and the like;
- polyhydric alcohols comprising from 2 to 20 carbon atoms and from 2 to 10 hydroxyl groups, in particular di-, tri- or tetrahydric alcohols, preferably those having 2 to 20 carbon atoms, such as ethylene glycol, propane-1,2- or -1,3-diol, butane-1,2- or -1,3-diol butene-1,4- or butine- 1,4-diol, hexane-1,6-diol, neopentyl glycol, dodecan-1,2-diol, 1,2,3-propanetriol, diethyleneglycol monoethyl ether, glycerol trimethylolpropane, and the like;
- glycols, such as glycerol, propylene glycol, 1,3-butylene glycol, dipropylene glycol, pentylene glycol, isoprene glycol, and the like;
- polymeric alcohols such as polyethylene glycols having a molecular weight not higher than 400, such as PEG 200, PEG 400, and ethers of polyethylene glycols such as tetrahydrofurfuryl alcohol PEG ether or methoxyPEG, and the like;
- fatty alcohols, such as for example stearyl, cetyl alcohols, and the like.

The **emulsifier-system** can be selected from any of the following classes of emulsifiers or surfactants :

### 1) Polyethoxylated Fatty Acids (PEG Fatty Acid esters)

Among the PEG-fatty acid monoesters, esters of lauric acid, oleic acid, and stearic acid are most useful, including PEG-8 laurate, PEG-8 oleate, PEG-8 stearate, PEG-9 oleate, PEG-10 laurate, PEG-10 oleate, PEG-12 laurate, PEG-12 oleate, PEG-15 oleate, PEG-20 laurate and PEG-20 oleate. Apart from these mono-esters, polyethylene glycol fatty acid diesters are also suitable for use as surfactants in the compositions of the present invention, for example PEG-20 dilaurate, PEG-20 dioleate, PEG-20 distearate, PEG-32 dilaurate and PEG-32 dioleate.

### 2) Polyethylene Glycol Glycerol Fatty Acid Esters

Suitable PEG glycerol fatty acid esters include PEG-20 glyceryl laurate, PEG-30 glyceryl laurate, PEG-40 glyceryl laurate, PEG-20 glyceryl oleate, and PEG-30 glyceryl oleate.

### 3) Alcohol-Oil Transesterification Products

A large number of surfactants of different degrees of hydrophobicity or hydrophilicity can be prepared by reaction of alcohols or polyalcohols with a variety of natural and/or hydrogenated oils. Most commonly, the oils used are castor oil or hydrogenated castor oil, or an edible vegetable oil such as corn oil, olive oil, peanut oil, palm kernel oil, apricot kernel oil, or almond oil. Preferred alcohols include glycerol, propylene glycol, ethylene glycol, polyethylene glycol, sorbitol, and pentaerythritol. Among these alcohol-oil transesterified surfactants, some typical surfactants are PEG-35 castor oil, PEG-40 hydrogenated castor oil, PEG-25 trioleate, PEG-60 corn glycerides, PEG-60 almond oil, PEG-40 palm kernel oil, PEG-50 castor oil, PEG-50 hydrogenated castor oil, PEG-8 caprylic/capric glycerides, PEG-6 caprylic/capric glycerides, PEG-5 hydrogenated castor oil, PEG-7 hydrogenated castor oil, PEG-9 hydrogenated castor oil, PEG-6 corn oil, PEG-6 almond oil, PEG-6 apricot kernel oil, PEG-6 olive oil, PEG-6 peanut oil, PEG-6 hydrogenated palm kernel oil, PEG-6 palm kernel oil, PEG-6 triolein, PEG-8 corn oil, PEG-20 corn glycerides, and PEG-20 almond glycerides. Also included as oils in this category of surfactants are oil-soluble vitamins, such as vitamin E. More specifically, TPGS which stands for tocopheryl PEG-100 succinate or D-α-tocopherol polyethylene glycol 1000 succinate is a particularly important surfactant.

### 4) Polyglycerized Fatty Acids

Polyglycerol esters of fatty acids are also suitable surfactants for the present invention, including polyglyceryl oleate, polyglyceryl-2 dioleate, polyglyceryl-10 trioleate, polyglyceryl-10 laurate, polyglyceryl-10 oleate and polyglyceryl-10 mono, dioleate and polyglyceryl polyricinoleates.

### 5) Propylene Glycol Fatty Acid Esters

Esters of propylene glycol and fatty acids include for example propylene glycol monolaurate, propylene glycol ricinoleate, propylene glycol monooleate, propylene glycol dicaprylate/dicaprate, and propylene glycol dioctanoate.

### 6) Mono- and Diglycerides

A large class of surfactants is the class of mono- and diglycerides, including glyceryl monooleate, glyceryl ricinoleate, glyceryl laurate, glyceryl dilaurate, glyceryl dioleate, glyceryl mono/dioleate, glyceryl caprylate/caprate, caprylic acid mono/diglycerides, and mono- and diacetylated monoglycerides.

### 7) Sterol and Sterol Derivatives

Sterols and derivatives of sterols are suitable surfactants for use in the present invention. Derivatives include the polyethylene glycol derivatives. Examples include cholesterol and PEG-24 cholesterol ether.

### 8) Polyethylene Glycol Sorbitan Fatty Acid Esters

A variety of PEG-sorbitan fatty acid esters are available and are suitable for use as surfactants in the present invention. Among the PEG-sorbitan fatty acid esters, possible surfactants include PEG-20 sorbitan monolaurate, PEG-20 sorbitan monopalmitate, PEG-20 sorbitan monostearate, and PEG-20 sorbitan monooleate.

### 9) Polyethylene Glycol Alkyl Ethers

Ethers of polyethylene glycol and alkyl alcohols are suitable surfactants for use in the present invention, including PEG-3 oleyl ether and PEG-4 lauryl ether.

### 10) Sugar Esters

Esters of sugars include sucrose monopalmitate and sucrose monolaurate.

### 11) Polyethylene Glycol Alkyl Phenols

Several PEG-alkyl phenol surfactants are available, and are suitable for use in the present invention, including the octyl and nonyl series known under the tradename Triton.

### 12) Polyoxyethylene-Polyoxypropylene Block Copolymers

The POE-POP block copolymers are a unique class of polymeric surfactants. The unique structure of the surfactants, with hydrophilic POE and hydrophobic POP moieties in well-defined ratios and positions, provides a wide variety of surfactants suitable for use in the present invention. These surfactants are available under various trade names, including Synperonic PE series (ICI) and Pluronic series (BASF). The generic term for these polymers is "poloxamer".

### 13) Sorbitan Fatty Acid Esters

Sorbitan esters of fatty acids are suitable surfactants for use in the present invention, including sorbitan monolaurate, sorbitan monopalmitate, sorbitan monooleate, sorbitan monostearate and sorbitan tristearate.

### 14) Lower Alcohol Fatty Acid Esters

Esters of lower alcohols and fatty acids are suitable surfactants for use in the present invention, for example ethyl oleate and isopropyl myristate or palmitate.

### 15) Ionic Surfactants

Ionic surfactants, including cationic, anionic and zwitterionic surfactants, are suitable surfactants for use in the emulisifier-system. This large group include fatty acid salts and bile salts, phospholipids, phosphoric acid esters, carboxylates, sulfates and sulfonates, and cationic surfactants, for example sodium oleate, sodium lauryl sulfate, sodium lauryl sarcosinate, sodium dioctyl sulfosuccinate, sodium cholate, sodium taurocholate, egg/soy lecithin, phosphatidyl ethanolamine and betaines. It will be appreciated by one skilled in the art, that any bioacceptable counterion may be used. For example, when sodium is given, other cation counterions can also be used, such as alkali metal cations or ammonium.

The **base-system** comprises one or more inorganic bases and/or one or more organic bases such as amines. The inorganic bases for use in the base-systems of the present inventions are selected from the group consisting of lithium hydroxide, sodium hydroxide, potassium hydroxide, calcium hydroxide, magnesium hydroxide, sodium carbonate, sodium bicarbonate, potassium carbonate, potassium bicarbonate, ammonium acetate, ammonium carbonate, sodium borate and mixtures thereof. The organic bases for use in the base-systems of the present invention are selected from the group consisting of methylamine, dimethylamine, trimethylamine, triethylamine, ethylamine, diethylamine, ethylenediamine, ethanolamine, N-methylglucamine (also known as 1-deoxy-1-(methylamino)-D-glucitol), amino acids and mixtures thereof.

The term "amino acid" as stated hereinabove is used in its broadest sense to mean the naturally occurring amino acids of general formula R-CH(COOH)-NH₂ (*i.e.* glycine, alanine, valine, leucine, isoleucine, methionine, proline, phenylanaline, tryptophan, serine, threonine, cysteine, tyrosine, asparagine, glutamine, aspartic acid, esters of aspartic acid, glutamic acid, esters of glutamic acid, lysine, arginine, and histidine) as well as non-naturally occurring amino acids, including amino acid analogs. Thus, reference herein to an amino acid includes, for example, naturally occurring proteogenic (L)-amino acids, as well as (D)-amino acids, chemically modified amino acids such as amino acid analogs, naturally occurring non-proteogenic amino acids such as norleucine, lanthionine or the like, and chemically synthesized compounds having properties known in the art to be characteristic of an amino can be incorporated into a protein in a cell through a metabolic pathway.

The base-system is typically present in an amount ranging from 0.5 to 3 mol equivalents with respect to the amount of anti-protozoal agent, more particularly the amount of base-system ranges from 2 to 3 mol equivalents with respect to the amount of anti-protozoal agent. Interestingly the amount of base-system is about 2 mol equivalents with respect to the amount of anti-protozoal agent. The expression "about 2 mol" means "2.0 ± 0.1 mol".

The anti-protozoal agent and the base-system may be combined by converting the anti-protozoal agent into its base addition salt form. For instance, diclazuril can be converted into its sodium hydroxide addition salt: "sodium diclazuril".

Preferred alcohol based solvent-systems are selected from ethanol, propylene glycol, PEG-200, PEG-400, and mixtures thereof.

Preferred emulsifier-systems are selected from TPGS, polyethyloxylated castor oil, and mixtures thereof.

Preferred base-systems are selected from sodium hydroxide, ethanolamine, triethanolamine, N-methyl-glucamine, and mixtures thereof.

One embodiment of the invention provides a composition comprising the anti-protozoal agent diclazuril wherein the alcohol based solvent-system comprises one or more alcohols selected from the group consisting of ethanol, polyethylene glycol, propylene glycol or mixtures thereof; to which a suitable base-system is added selected from the group consisting of sodium hydroxide, ethanolamine, N-methylglucamine, and mixtures thereof; and an emulsifier-system selected from the group consisting of TPGS, polyethyloxylated castor oil (*e.g*. under the trade name Cremophor^{™}), and mixtures thereof; formulated for oral or parenteral administration for the treatment of protozoal infections in man or in animals.

Yet another embodiment of the invention provides a composition formulated for oral or transdermal administration and possessing a defined rheological and/or bio-adhesive character due to the addition of adhesive and/or thickening and/or visco-modulating agents. Such suitable adhesive and/or thickening and/or visco-modulating agents may be of those known and employed in the art, including for example pharmaceutically acceptable polymeric materials and inorganic thickening agents, and mixtures thereof, for example of the following types :
- cellulose and cellulose derivatives including alkylcelluloses, hydroxyalkylcelluloses such as hydroxypropyl-methyl-cellulose (hypromellose), acetylated celluloses, and salts thereof as well as mixtures ;
- polyvinylpyrrolidones and vinylpyrrolidone co-polymers ;
- polyethylene glycols, polyethylene oxides and derivatives ;
- carbomers ;
- polysaccharide-type polymers such as alginates, polydextroses, carrageenan, tragacanth, xanthan and acacia gums ;
- inorganic thickening agents such as silicates (including silicon dioxide products and derivatives) and related magnesium- and aluminium complexes including bentonite and atapulgite.

The compositions may also include one or more further ingredients like anti-oxidants (e.g. vitamin C, ascorbyl palmitate and other vitamin C derivatives, butyl hydroxyl anisole (BHA), butyl hydroxyl toluene (BHT), antimicrobial agents, flavouring and colouring agents, and so forth.

The relative proportion of ingredients in the compositions of the invention will, of course, vary considerably depending on the particular type of composition concerned. Determination of workable proportions in any particular instance will generally be within the capability of the man skilled in the art.

Also provided by the invention is a method of preparation of these compositions for use in the treatment of parasitic infections.

The quantity of the anti-protozoal agent in the compositions of the present invention will be so that an effective antiprotozoal effect is obtained. In particular it is contemplated that such compositions comprise the anti-protozoal agent in a range from 0.01 % to 10 % (w/v), in particular from 0.1 % to 5 % (w/v), more particular from 0.5 % to 2 % (w/v). In many instances the antiprotozoal compositions to be used directly can be obtained from concentrates, such as e.g. emulsifiable concentrates, suspension concentrates, or soluble concentrates, upon dilution with aqueous or organic media, such concentrates being intended to be covered by the term composition as used in the definitions of the present invention. Such concentrates can be diluted to a ready to use mixture in a tank shortly before use.

It is contemplated that the compositions of the invention can be formulated for any oral, parenteral and transdermal administration. It is specifically contemplated that the intravenous, intramuscular, intranasal and subcutaneous routes of parenteral administration can be utilized for administration of the formulation of the invention. Specific formulations of the invention can include solutions, as well as semi-solid formulations like gels, pastes and ointments, sustained release preparations, patches and the like.

The compositions according to the present invention are suitable for controlling parasitic protozoa which occur in livestock management and livestock breeding in useful, breeding, and pet animals. They are moreover active against all or individual stages of development of the pests and against resistant and normally sensitive strains. The intention of the control of the parasitic protozoa is to reduce disease, deaths and reductions in performance (for example in the production of meat, milk, wool, hides, eggs, honey etc.) so that the use of the active compounds makes more economic and livestock management possible.

The parasitic protozoa include:
- Mastigophora (Flagellata) such as, for example Trypanosomatidae, for example Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, such as, for example, Trichomonadidae, for example Giardia lamblia, G. canis.
- Sarcomastigophora (Rhizopoda) such as Entamoebidae, for example Entamoeba histolytica, Hartmanellidae, for example Acanthamoeba sp., Hartmanella sp.
- Apicomplexa (Sporozoa) such as Eimeridae, for example Eimeria acervulina, E. adenoides, E. alabahmensis, E. anatis, E. anseris, E. arloingi, E. ashata, E auburnensis, E. bovis. E. brunetti, E. canis, E. chinchillae, E. clupearum, E columbae, E. contorta, E. crandalis, E. debliecki, E. dispersa, E. ellipsoidales, E falciformis, E. faurei, E. flavescens, E. gallopavonis, E. hagani, E. intestinalis, E iroquoina, E. irresidua, E. labbeana, E. leucarti, E. magna, E. maxima, E. media, E. meleagridis, E. meleagrimitis, E. mitis, E. necatrix, E. ninakohlyakimovae, E ovis, E. parva, E. pavonis, E. perforans, E. phasani, E. piriformis, E. praecox, E residua, E. scabra, E. spec., E. stiedai, E. suis, E. tenella E. truncata, E. truttae, E zuemii, Globidium spec., Isospora belli, I. canis, I. felis, I. ohioensis, I. rivolta, I spec., I. suis, Cystisospora spec., Cryptosporidium spec. such as Toxoplasmadidae, for example Toxoplasma gondii, such as Sarcocystidae, for example Sarcocystis bovicanis, S. bovihominis, S. ovicanis, S. ovifelis, S. spec., S. suihominis such as Leucozoidae, for example Leucozytozoon simondi, such as plasmodiidae, for example plasmodium berghei, P. falciparum, P. malariae, P. ovale, P. vivax, P spec., such as Piroplasmea, for example Babesia argentina, B. bovis, B. canis, B spec. Theileria parva, Theileria spec., such as Adeleina, for example Hepatozoon canis, H. spec.
- Neospora spp. and Neospora caninum.

The useful and breeding livestock include mammals such as, for example, cattle, horses, sheep, pigs, goats, camels, water buffalo, donkeys, rabbits, fallow deer, reindeer, fur-bearing livestock such as, for example, mink, chinchilla, raccoon, birds such as, for example, poultry, chickens, geese, turkeys, ducks, pigeons, bird species for keeping at home and in zoos. Pet animals include dogs and cats.

Both prophylactic and therapeutic use is possible.

The compositions according to the present invention are particularly suitable for combating protozoal diseases such as coccidioses and similar diseases in a large number of mammals, such as equidae (horses, donkeys, etc.), ruminants (cattle, sheep, goats, camels or related species, etc.), poultry, pigs, dogs, cats, rabbits, rodents or other mammals. Coccidioses and similar diseases are to be understood as meaning infections with infective stages of species of various genera, such as, for example, Eimeria, Isospora, Castoisospora, Sarkocystis, Toxoplasma, Neospora or Cryptosporidae.

The compositions of the present invention are also suitable in the treatment of EPM (Equine Protozoal Myeloencephalitis). EPM is an infectious neurological disease of horses caused by Sarcocystis neurona.

The following examples are intended to illustrate and not to limit the scope of the present invention in all its aspects.

### Example 1

Sodium diclazuril solid drug substance was obtained from Janssen Pharmaceutica N.V. TPGS was purchased from Eastman Chemical Co. in the form of a natural d-alpha-tocopherol polyethylene glycol-1000-succinate, labelled NF quality. Ethanol was purchased from Belgalco N.V. and was labelled 94% purity.

First, the mixture of ethanol (60% w/w) and TPGS (40% w/w) mixture was prepared in a large quantity (500 ml) by transferring 168 g TPGS into a 500 ml-flask, adding up the volume with ethanol and heating at 60°C till complete solubilisation of TPGS. Formulations were prepared by weighing different amounts of sodium diclazuril, ranging between 0.05 and 0.75 gram, and adding ethanol-TPGS solution till 100 ml. Sonication and heating at 60°C for 10 minutes was performed.

The resultant solutions were all clear and slightly yellow, with nominal concentrations ranging between 0.05 and 0.75% (w/v) sodium diclazuril. Higher concentrations of sodium diclazuril can be solubilised.

This formulation was orally administered to mice using gavage and compared with two commercially available formulations : a suspension (COM1) and an aqueous alkaline, solution (COM2). A 5 mg/kg body weight dose of each formulation was administered. There were six mice per formulation. At 30, 60 and 120 minutes after administration, blood samples were collected into EDTA-containing test tubes from 2 mice per formulation. The tubes were centrifuged during 10 minutes at 500 x g. The plasma samples from 2 mice per formulation per time were pooled into sterile Eppendorf vials and analysed using LC-MS/MS. Results are presented in Table 1.

**Table 1: Diclazuril plasma concentration after oral administration to mice**

| **Composition** | **Time (minutes)** | **Diclazuril (µg/ml)** | **Diclazuril AUC₀₋₂ₕᵣₛ (µg.min/ml)** |
|---|---|---|---|
| | 30 | 2.7 | |
| COM1 | 60 | 2.2 | 249 |
| | 120 | 2.3 | |
| | 30 | 3.4 | |
| COM2 | 60 | 2.8 | 291 |
| | 120 | 2.1 | |
| | 30 | 6.3 | |
| Example 1 | 60 | 6.0 | 636 |
| | 120 | 5.9 | |

The UC_{0-2 hours} after administration was at least 2 times higher in the present formulation than in the commercial suspension and aqueous solution. AUC₀₋₂ hours means the area under the plasma concentration-time curve measured between 0 and 2 hours after administration of the test formulation.

COM1 is a commercial diclazuril suspension known under the tradename Vecoxan^{™} having the following composition :

| | | |
|---|---|---|
| Diclazuril | 2.5 mg | 0.25% |
| Microcrystalline cellulose and Carboxymethylcellulose sodium | 12 mg | 1.2% |
| Methyl parahydroxybenzoate | 1.8 mg | 0.18% |
| Polysorbate 20 | 0.5 mg | 0.05% |
| Propyl prahydroxybenzoate | 0.2 mg | 0.02% |
| Citric acid monohydrate | 1 mg | 0.1% |
| Sodium hydroxide | q.s. ad pH | q.s. ad pH |
| Purified water | q.s. ad 1 ml | q.s. ad 100% |

The formula is expressed in mg/ml and in % w/v.

COM2 is an aqueous alkaline solution comprising 0.5% diclazuril known under the tradename Nuoqiu^{™} and commercially available from Shandong Luxi Animal Medicine Share Company Ltd, Qike Shandong 251100, People's Republic of China. The exact composition is unknown.

### Example 2

A solution consisting of ethanol (25% w/w), TPGS (33% w/w) and triethanolamine (42% w/w) was prepared by mixing the compounds and heating at 60°C for 10 minutes. To this mixture, diclazuril was added, sonicated and heated at 60°C for 10 minutes to obtain a solution of 0.25%w/v; higher concentrations (e.g. 1% w/v) were also prepared.

The formulation as prepared in Example 2 was administered to mice (identical methodology as described in Example 1) and following results were obtained:

**Table 2: Diclazuril plasma concentration after oral administration to mice**

| **Composition** | **Diclazuril (µg/ml)** | **Diclazuril AUC₀₋₂ₕᵣₛ (µg.min/ml)** |
|---|---|---|
| | 4.1 | |
| Example 2 | 5.8 | 608 |
| | 7.5 | |

### Example 3

A solution consisting of ethanol (29.85 % w/w), PEG 400 (29.85 % w/w), TPGS (39.80 % w/w) and ethanolamine (0.50 % w/w) was prepared by mixing the compounds and heating at 60°C for 10 minutes. Diclazuril was added and easily solubilised to obtain a final concentration of 0.5% w/v.

### Example 4

A solution consisting of ethanol (29.80% w/w), PEG 400 (29.80% w/w), TPGS (39.75% w/w) and N-methylglucamine (0.65% w/w) was prepared by mixing these excipients and heating at 60°C for 10 minutes. Diclazuril was added and easily solubilised to obtain a final concentration of 0.5% w/v.

Intrinsic stability was investigated using HPLC analysis of samples stored at stressed (50°C), accelerated (40°C) and long-term (25°C and 5°C) storage conditions. Composition no. 5, for example, stored for 2 months at 50°C still showed a diclazuril assay value of 92.5%, with little keto-degradation product observed. The aspect of the formulation still was clear and slightly yellow, i.e. similar to the aspect immediately after preparation.

### Example 5

A solution consisting of ethanol (39.74 % w/w), PEG 400 (39.74 % w/w), TPGS (19.87% w/w) and N-methylglucamine (0.65% w/w) was prepared by mixing the compounds and heating at 60°C for 10 minutes. Diclazuril was added and easily solubilised to obtain a final concentration of 0.5% w/v.

### Example 6

A solution consisting of ethanol (44.71% w/w), PEG 400 (44.71% w/w), TPGS (9.93% w/w) and N-methylglucamine (0.65% w/w) was prepared by mixing the compounds and heating at 60°C for 10 minutes. Diclazuril was added and easily solubilised to obtain a final concentration of 0.5% w/v.

Mice were orally administered a single amount of one of the four previously described formulations (example 3 to 6) directly into the gastro-intestinal system using gavage. A 5 mg/kg body weight dose of each formulation was administered. There were six mice per formulation.

At 30, 60 and 120 minutes after administration, blood was collected into EDTA-containing test tubes from 2 mice per formulation. The tubes were centrifuged during 10 minutes at 500 x g. The plasma samples from 2 mice per formulation per time were pooled into sterile Eppendorf vials and analysed using LC-MS/MS. The results are given in Table 3.

**Table 3: Diclazuril plasma concentrations after oral administration to mice**

| **Composition** | **Time (minutes)** | **Diclazuril (µg/ml)** | **Diclazuril AUC₀₋₂ₕᵣₛ (µg.min/ml)** |
|---|---|---|---|
| | 30 | 7.60 | |
| Example 3 | 60 | 12.43 | 968 |
| | 120 | 6.02 | |
| | 30 | 7.73 | |
| Example 4 | 60 | 13.86 | 1176 |
| | 120 | 10.69 | |
| | 30 | 8.75 | |
| Example 5 | 60 | 6.56 | 763 |
| | 120 | 6.83 | |
| | 30 | 7.00 | |
| Example 6 | 60 | 6.73 | 690 |
| | 120 | 5.92 | |

### Example 7

A solution consisting of ethanol (29.80% w/w), PEG 400 (29.80% w/w), TPGS (39.75% w/w) and N-methyl-glucamine (0.65% w/w) was prepared by mixing the compounds and heating at 60°C for 10 minutes. Diclazuril was added and easily solubilised to obtain a final concentration of 0.25% w/v.

Example 7 formulation was administered to turkeys by gavage at a dosage of 2 ml/kg body weight (total dose: 5 mg diclazuril/kg body weight).

Blood was collected from 6 turkeys at 2, 4 and 8 hours after administration and the diclazuril content of plasma was analysed using LC-MS. Results are shown in Table 4.

**Table 4: Diclazuril plasma concentrations after oral administration to turkeys**

| **Formulation** | **Time (hours)** | **Diclazuril (µg/ml)** | **AUC₀₋₈ₕ (µg.min/ml)** |
|---|---|---|---|
| | 2 | 7.37 | |
| Example 7 | 4 | 10.81 | 4040 |
| | 8 | 10.08 | |

The advantages of this approach are manifold: in the first place effective plasma concentrations can be attained within a short time period after administration, which can lead to shorter treatment periods. In the second place, the required plasma concentrations can be attained by the administration of smaller quantities of diclazuril, which may lead to substantial savings on drug costs. Thirdly, increased plasma concentrations are rapidly attained, leading to rapid entry of diclazuril into infected tissues. Fourthly, the formulations are stable when stored below 25°C: the amount of the keto-degradation product, the major degradation product of diclazuril as found in, for instance, alkaline aqueous-based formulations, can be maintained below 3% for several months to years in these formulations.

## Claims

1. A composition comprising the anti-protozoal agent diclazuril dissolved in a mixture comprising
a) an alcohol based solvent-system,
b) an emulsifier-system, and
c) a base-system;
wherein the base-system is present in an amount ranging from 0.5 to 3 mol equivalents with respect to the amount of anti-protozoal agent.

2. A composition according to claim 1 wherein
a) the alcohol based solvent-system is selected from lower alcohols comprising from 1 to 8 carbon atoms, polyhydric alcohols comprising from 2 to 20 carbon atoms and from 2 to 10 hydroxyl groups, glycols, polyethylene glycols, fatty alcohols, and mixtures thereof;
b) the emulsifier-system is selected from polyethoxylated fatty acids, polyethylene glycol glycerol fatty acid esters, alcohol-oil transesterification products, polyglycerized fatty acids, propylene glycol fatty acid esters, mono- and diglycerides, sterol and sterol derivatives, polyethylene glycol sorbitan fatty acid esters, polyethylene glycol alkyl ethers, sugar esters, polyethylene glycol alkyl phenols, polyoxyethylene-polyoxypropylene block copolymers, sorbitan fatty acid esters, lower alcohol fatty acid esters, ionic surfactants, and mixtures thereof;
c) the base-system is an inorganic base selected from lithium hydroxide, sodium hydroxide, potassium hydroxide, calcium hydroxide, magnesium hydroxide, sodium carbonate, sodium bicarbonate, potassium carbonate, potassium bicarbonate, ammonium acetate, ammonium carbonate, and mixtures thereof; and/or an organic base selected from the group consisting of methylamine, dimethylamine, trimethylamine, triethylamine, ethylamine, diethylamine, ethylenediamine, ethanolamine, N-methylglucamine, and mixtures thereof.

3. A composition according to any of claims 1 to 2 wherein the alcohol based solvent-system comprises one or more alcohols selected from ethanol, propylene glycol, PEG-200, PEG-400 or mixtures thereof.

4. A composition according to any of claims 1 to 3 wherein the emulsifier-system comprises one or more emulsifiers selected from TPGS, polyethyloxylated castor oil, and mixtures thereof.

5. A composition according to any of claims 1 to 4 wherein the base-system comprises one or more bases selected from sodium hydroxide, ethanolamine, triethanylamine, N-methylglucamine, and mixtures thereof.

6. A composition according to any of claims 1 to 2 wherein the alcohol based solvent-system consists of a mixture of ethanol and PEG 400, the emulsifier-system consists of TPGS, and the base-system consists of N-methylglucamine.

7. A composition according to any of claims 1 to 6 wherein the base-system is present in an amount ranging from 2 to 3 mol equivalents with respect to the amount of the anti-protozoal agent diclazuril.

8. A composition according to any of claims 1 to 4 wherein the anti-protozoal agent diclazuril and the base-system are combined by converting diclazuril into its base addition salt form.

9. Use of a composition as claimed in any of claims 1 to 8 for the manufacture of a medicament for the treatment of protozoal infections.

10. Use according to claim 9 wherein the protozoal infection is Equine Protozoal Myeloencephalitis.

## Patentansprüche

1. Verbindungen enthaltend, das Antiprotozoenmittel Diclazuril gelöst in einer Mischung, umfassend
a) ein Lösungsmittelsystems auf Alkoholbasis
b) ein Emulgatorsystem und
c) ein Basensystem;
wobei das Basensystem in einer Menge im Bereich von 0,5-3 Moläquivalenten, bezogen auf die Menge an Antiprotozoenmittel, vorhanden ist.

2. Zusammensetzungen nach Anspruch 1, wobei
a) das Lösungsmittelsystem auf Alkoholbasis ausgewählt ist aus niederen Alkoholen mit 1-8 Kohlenstoffatomen, mehrwertigen Alkoholen mit 2 bis 20 Kohlenstoffatomen und 2 bis 10 Hydroxylgruppen, Glykolen, Polyethylenglykolen, Fettalkoholen und Mischungen davon;
b) das Emulgatorsystem ausgewählt ist aus polyethoxylierten Fettsäuren, Polyethylenglykolglycerinfettsäureestern, Alkohol-Ö1-Umesterungsprodukten, polyglycerinierten Fettsäuren, Propylenglykolfettsäureestern, Mono- und Diglyceriden, Sterol und Sterolderivaten, Polyethylenglykolsorbitanfettsäureestern, Polyethylenglykolalkylethern, Zuckerestern, Polyethylenglykolalkylphenolen, Polyoxyethylen-Polyoxypropylen-Blockcopolymeren, Sorbitanfettsäureestern, Niederalkoholfettsäureestern, ionischen Tensiden und Mischungen davon;
c) es sich bei dem Basensystem um eine anorganische Base ausgewählt aus Lithiumhydroxid, Natriumhydroxid, Kaliumhydroxid, Calciumhydroxid, Magnesiumhydroxid, Natriumcarbonat, Natriumhydrogencarbonat, Kaliumcarbonat, Kaliumhydrogencarbonat, Ammoniumacetat, Ammoniumcarbonat und Mischungen davon und/oder eine organische Base ausgewählt aus der aus Methylamin, Dimethylamin, Trimethylamin, Triethylamin, Ethylamin, Diethylamin, Ethylendiamin, Ethanolamin, N-Methylglucamin und Mischungen davon bestehenden Gruppe handelt.

3. Zusammensetzungen nach Anspruch 1 oder 2, wobei das Lösungsmittelsystem auf Alkoholbasis ein oder mehrere Alkohole ausgewählt aus Ethanol, Propylenglykol, PEG-200, PEG-400 oder Mischungen davon enthält.

4. Zusammensetzungen nach einem der Ansprüche 1 bis 3, wobei das Emulgatorsystem einen oder mehrere Emulgatoren ausgewählt aus TPGS, polyethoxyliertem Rizinusöl und Mischungen davon enthält.

5. Zusammensetzungen nach einem der Ansprüche 1 bis 4, wobei das Basensystem ein oder mehrere Basen ausgewählt aus Natriumhydroxid, Ethanolamin, Triethanolamin, N-Methylglucamin und Mischungen davon enthält.

6. Zusammensetzungen nach Anspruch 1 oder 2, wobei das Lösungsmittelsystem auf Alkoholbasis aus einer Mischung aus Ethanol und PEG 400 besteht, das Emulgatorsystem aus TPGS besteht und das Basensystem aus N-Methylglucamin besteht.

7. Zusammensetzungen nach einem der Ansprüche 1 bis 6, wobei das Basensystem in einer Menge im Bereich von 2 bis 3 Moläquivalenten, bezogen auf die Menge an Antiprotozoenmittel Diclazuril, vorliegt;

8. Zusammensetzungen nach einem der Ansprüche 1 bis 4, wobei das Antiprotozoenmittel Diclazuril und das Basensystem kombiniert werden, indem man Diclazuril in seine Basenadditionssalzform umwandelt.

9. Verwendung einer Zusammensetzung nach einem der Ansprüche 1-8 zur Herstellung eines Medikaments zur Behandlung von Protozoeninfektionen.

10. Verwendung nach Anspruch 9, wobei es sich bei der Protozoeninfektion um die Equine Protozoäre Myeloenzephalitis handelt.

## Revendications

1. Composition comprenant un agent antiprotozoaire, le diclazuril, dissous dans un mélange comprenant
a) un système de solvant à base d'alcool,
b) un système d'émulsifiants, et
c) un système de bases ;
**caractérisée en ce que** le système de bases est présent dans une quantité allant de 0,5 à 3 équivalents molaires par rapport à la quantité d'agents antiprotozoaires.

2. Composition selon la revendication 1, **caractérisée en ce que**
a) le système de solvant à base d'alcool est choisi parmi les alcools inférieurs comprenant de 1 à 8 atomes de carbone, les alcools polyhydriques comprenant de 2 à 20 atomes de carbone et de 2 à 10 groupements hydroxy, les glycols, les polyéthylèneglycols, les alcools gras, et les mélanges de ceux-ci ;
b) le système d'émulsifiants est choisi parmi les acides gras polyéthoxylés, les esters d'acides gras et de polyéthylèneglycol et de glycérol, les produits de transestérification alcool-huile, les acides gras polyglycérolés, les esters d'acides gras et de propylèneglycol, les mono- et diglycérides, le stérol et les dérivés du stérol, les esters d'acides gras de polyéthylèneglycol et de sorbitane, les alkyléthers de polyéthylèneglycol, les esters de sucres, les alkylphénols de polyéthylèneglycol, les copolymères séquencés polyoxyéthylène-polyoxypropylène, les esters d'acides gras et de sorbitane, les esters d'acides gras et d'alcools inférieurs, les tensioactifs ioniques, et les mélanges de ceux-ci ;
c) le système de bases est une base minérale choisie parmi l'hydroxyde de lithium, l'hydroxyde de sodium, l'hydroxyde de potassium, l'hydroxyde de calcium, l'hydroxyde de magnésium, le carbonate de sodium, le bicarbonate de sodium, le carbonate de potassium, le bicarbonate de potassium, l'acétate d'ammonium, le carbonate d'ammonium et les mélanges de ceux-ci ; et/ou une base organique choisie dans le groupe constitué de méthylamine, de diméthylamine, de triméthylamine, de triéthylamine, d'éthylamine, de diéthylamine, d'éthylènediamine, d'éthanolamine, de N-méthylglucamine, et de mélanges de celles-ci.

3. Composition selon l'une ou l'autre des revendications 1 et 2, **caractérisée en ce que** le système de solvant à base d'alcool comprend un ou plusieurs alcools choisis parmi l'éthanol, le propylèneglycol, le PEG-200, le PEG-400 ou les mélanges de ceux-ci.

4. Composition selon l'une quelconque des revendications 1 à 3, **caractérisée en ce que** le système d'émulsifiants comprend un ou plusieurs émulsifiants choisis parmi le TPGS, l'huile de ricin polyéthoxylée, et les mélanges de ceux-ci.

5. Composition selon l'une quelconque des revendications 1 à 4, **caractérisée en ce que** le système de bases comprend une ou plusieurs bases choisies parmi l'hydroxyde de sodium, l'éthanolamine, la triéthanolamine, la N-méthylglucamine, et les mélanges de ceux-ci.

6. Composition selon l'une ou l'autre des revendications 1 et 2, **caractérisée en ce que** le système de solvant à base d'alcool est constitué d'un mélange d'éthanol et de PEG 400, le système d'émulsifiants est constitué de TPGS, et le système de bases est constitué de N-méthylglucamine.

7. Composition selon l'une quelconque des revendications 1 à 6, **caractérisée en ce que** le système de bases est présent dans une quantité allant de 2 à 3 équivalents molaires par rapport à la quantité d'agent antiprotozoaire, le diclazuril.

8. Composition selon l'une quelconque des revendications 1 à 4, **caractérisée en ce que** l'agent antiprotozoaire, le diclazuril et le système de bases sont combinés en transformant le diclazuril en sa forme de sel d'addition de bases.

9. Utilisation d'une composition selon l'une quelconque des revendications 1 à 8, pour la fabrication d'un médicament destiné au traitement d'infections à protozoaires.

10. Utilisation selon la revendication 9, **caractérisée en ce que** l'infection à protozoaires est la myéloencéphalite équine à protozoaires.
